# EUROPEAN PATENT APPLICATION

(11) **EP 4 079 275 A1**
(43) Date of publication of application: **26.10.2022**
(21) Application number: 21169498.9
(22) Date of filing: 20.04.2021
(51) Int. Cl.: A61F 13/511, A61F 13/513, A61F 13/512, D04H 1/4258, D04H 1/495

(54) **ABSORBENT HYGIENE PRODUCTS WITH APERTURED PLANT MATERIAL BASED TOPSHEET**

(71) Applicant: W. Pelz GmbH & Co. KG, 23812 Wahlstedt/Holstein (DE)
(72) Inventor: RÖTTGER, Henning, 24568 Kaltenkirchen (DE); MAYER, Dominik, 93339 Riedenburg (DE); BERNT, Ingo, 93053 Regensburg (DE)
(74) Representative: Plischke, Manfred

(57) **Abstract**

The present invention relates to absorbent articles, in particular to such articles comprising plant based fibrous materials useful as topsheet materials that are particularly adapted for urine handling.

## Description

### Field of the invention

The present invention relates to absorbent articles, in particular to such articles comprising plant based fibrous materials useful as topsheet materials, that are particularly adapted for urine handling.

### Background

There is an increasing consumer demand for absorbent hygiene products with reduced amount of plastic being replaced by sustainable natural and preferably plant-based materials. Thus, it is known to use plant based fibrous webs based on cotton or regenerated cellulose, such as known as Viscose / Rayon or Lyocell fibers spunlaced or - as interchangeable term - hydroentangled topsheet materials in "bio-products" replacing conventional polymer (mostly PP) based nonwoven materials.
However, for applications of webs coming into direct contact with the consumer's skin such as topsheets, the intrinsic hydrophilic nature of such natural material based fibres results in an unsatisfactory surface dryness of the web upon loading with higher amounts of liquid.
A known approach uses unbleached or semi bleached cotton in spunlaced topsheet materials. Due to the content of natural waxes, these fibres are hydrophobic resulting in a water repellent topsheet, as further described in EP1764077A2 in the context of cosmetic pads.
Kelheim Fibres, Germany, has developed a hydrophobic viscose fibre using plant-based additives, as described in EP2931952 and marketed under the trade designation "Olea". Water repellent nonwoven materials have been produced using 100% Olea fibres in a spunlace process. However, as webs with 100% of such fibers provide poor liquid permeability, Kelheim Fibers have also disclosed to blend such fibers with bleached cotton or viscose fibres. Different top-sheet materials made of blends of Olea fibres and viscose fibres have been tested in combination with different acquisition layer materials revealing the potential to design catamenial feminine hygiene products with good intake time and rewet for menses (2nd International Conference on Cellulose Fibres 2-3 February 2021, Online Event of nova-Institute, Hürth, Germany). However, the described application was for menstrual products with low liquid loads, as reflected in the shown performance data according to standard tests with 3x5 ml test fluid. However, such performance parameters are not representative for the use in incontinence products with significantly higher urine loads, as reflected in typical test conditions of e.g. 3 x 50 ml of 0.9 % NaCl solution.

It is also known to use topsheets with a pattern of apertures. One approach in this direction uses a polymeric film material with a pattern of apertures. Fibrous webs are typically made from synthetic polymeric material, such as polypropylene, and adapted to the intended use. When aiming at urine handling, such topsheets are treated with a hydrophilizing agent, rendering these webs permanently or non-permanently hydrophilic so as to ease liquid reception. When such webs are intended for feminine hygiene application with typically low fluid load, or for dealing with low viscosity faeces (e.g. US5342388; 1994, P&G, Roe) these webs are kept at the intrinsic material hydrophobicity.
In CN206151698U a natural raw cotton diaper is described, comprising a surface layer, an absorbing layer and an anti-leakage layer, wherein the surface layer is natural raw cotton spun-laced non-woven fabric. The surface layer is provided with multiple openings by mechanical punching, e.g. stamping, rolling. The surface layer retains the original pectin and wax of the raw cotton, thereby exhibiting its natural water-repellent performance. However, none of these approaches provides for plant based materials a suitable balance of liquid intake or acquisition and rewet for articles intended to be used for higher urine loads, such as adult incontinence articles.
Thus, the present invention aims at providing such articles, which exhibit good liquid acceptance as well as good surface dryness.

### Summary

The present invention is an absorbent article comprising a fibrous topsheet, a backsheet, and an absorbent core positioned there between, exhibiting an absorbent capacity of at least 150ml of synthetic urine according to the Centrifuge Capacity Test. The topsheet comprises at least about 90 w-%, preferably at least about 95 w-%, and even more preferably consists essentially of plant based fibers, preferably cellulose based, more preferably of regenerated cellulose. The fibers are hydrophobically treated by incorporating a hydrophobic substance into the fiber matrix, which may be selected from the group consisting of alkylketene dimers, alkenylketene dimers, alkylsuccinic anhydrides, alkenylsuccinic anhydrides, alkylglutaric anhydrides, alkenylglutaric anhydrides, alkyl isocyanates, alkenyl isocyanates, fatty acid anhydrides and mixtures thereof.

The topsheet exhibits land area and a pattern, optionally comprising sub-patterns, of apertures, satisfying at least one of the following features selected from the group consisting of
- the apertures exhibiting an elongated shape, wherein the longer extension is at least 1.5 times the smaller extension;
- the apertures exhibiting a smaller extension of at least about 0.25 mm;
- the apertures exhibiting a lager extension of at least about 1.00 mm;
- the aperture area being at least about 0. 1 mm²;
- the aperture area being less than about 15.0 mm²,
- the apertures covering at least about 1 % of the total area of the topsheet;
- the apertures covering less than about 50% of the total area of the topsheet;
- an average Interaperture distance of more than about 1mm;
- an average Interaperture distance of less than about 20 mm.
Further, the topsheet exhibits a hydrophobicity according to the Single Load Drop Test Preferably, the plant based fibers comprise regenerated cellulose based on cotton, as may be based on the Lyocell process.
The topsheet may comprise hydrophilic fibers, preferably regenerated cellulose fibers, in an amount of less than about 10 w-%, preferably less than about 5 w-%.
Optionally, the topsheet may comprise a non-permanent hydrophilization agent, or a fragrance solution.
Preferably, the topsheet exhibits only an initial hydrophilicity and not a permanent hydrophilicity according to the Washed Substrate Drop Test.
An absorbent article preferably exhibits a rewet of less than about 10 g, preferably less than about 5 g, and more preferably less than about 3 g, and most preferably of less than about 2 g. whilst exhibiting an acquisition time of less than about 200 s, according to the acquisition and rewet test using 3 x 150 ml of 0.9 % saline solution.

### Brief description of the Figures (yet to be finalized)

Fig. 1 depicts schematically the layers of an article according to the present invention.
Fig. 2 depicts schematically a top view of an apertured topsheet suitable for the present invention.
Fig. 3 shows a diagram with test results on comparative samples and samples according to the present invention.

Figures are schematic and not to scale. Same numerals depict same or equivalent features or elements.

### Detailed description

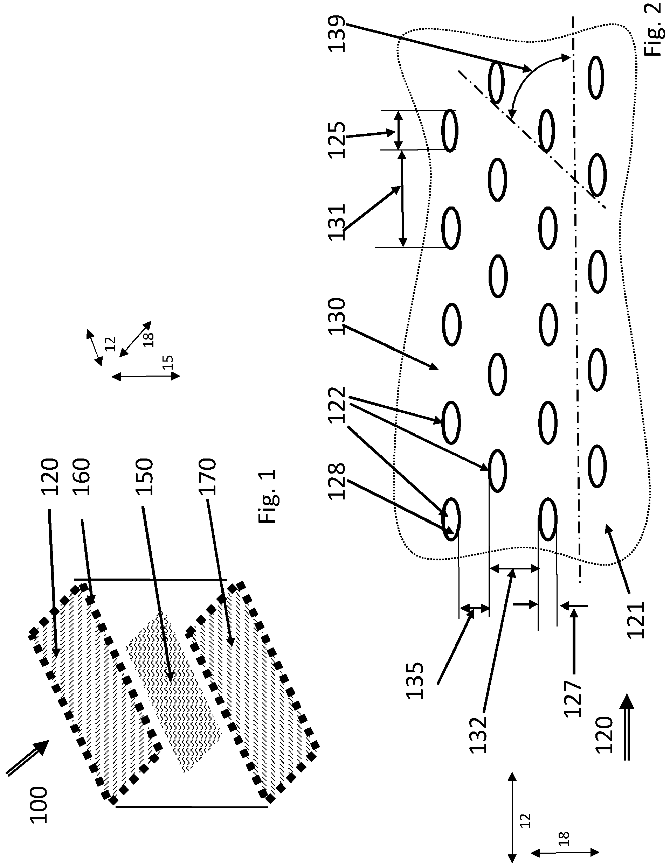

Absorbent articles according to the present invention comprise an absorbent core that is adapted to receive moderate to high amounts of liquid, especially urine.
Such articles are typically intended for use as adult incontinence articles, including Feminine Hygiene articles, though use for babies or toddlers may be included.
Such articles should exhibit an absorbent capacity, such as determined by centrifuge capacity test, of more than about 150 ml, or more than about 300 ml, or more than about 450 ml or even more than about 600 ml of saline.
Such articles may be worn on the lower torso of a wearer, either in a "closed pants style" or as "open diapers" executed with closure means, such as mechanical fastener or adhesive tapes. Such articles may also be absorbent pads, that are worn in combination with other means such as net pants or underwear. Without limiting the present invention to such pads, Fig. 1 depicts schematically such an article 100 comprising a topsheet 120, a backsheet 170 and an absorbent core 150 there between, whereby the topsheet 120 and the backsheet 170 are connected to each other, e.g. by a circumferential bond, here shown exemplarily as bond line 160. The article exhibits a length or x-directional 12 extension as may be aligned with the machine direction when produced, further a width or y-directional extension 18, as may be aligned with the cross-machine direction when produced, and a thickness 15 or height perpendicular thereto.
In order to prevent wetting of clothing or bedding once the article is loaded, the liquid impermeable backsheet 170 as may be a polymeric or a natural based film or water repellent fibrous material. The topsheet 120 is intended to be in contact with the skin of the wearer, and thus should be comfortable and soft, but also allow liquid to pass through to the absorbent core 150 upon a liquid load, but should minimize rewetting of liquid back to the skin of a wearer.
It should be noted that within the present context the terms over / under refer to a direction along gravity as indicated in Fig. 1. However, the skilled person will readily realize, that an article can be turned over and then a backsheet could be seen to be positioned over the core and topsheet.
In order to achieve a dry surface after exposure to liquid, it is important to have a driving force directing the liquid from the topsheet to the core. The flow of liquid is driven by the capillary force of the porous structure of topsheet, acquisition/distribution layer (ADL), if present, and absorbent core, which should increase from topsheet via ADL to the core, even against gravity. The capillary force may be described by the Young-Laplace equation according to which the capillary force increases for aqueous systems with decreasing pore diameter and increasing hydrophilicity. Plant based materials, such as cellulose exhibit very high water affinity (hydrophilicity) and retain liquid not only in the interstitial voids of the web but also within the fibers. Thus, an underlying ADL should exhibit sufficient void space in its inter-fiber pores so as to accommodate liquid gushes and to allow lateral flow to distribute the liquid. Further, the ADL should exhibit a suction capability which is lower than the one of the underlying absorbent core to allow emptying of the inter-fiber pores before application of the next gush load.
Henceforth, in order to not overly compromise rewetting, articles with conventional topsheets made from or with plant based materials, especially fibers, can only handle small to moderate amounts of liquid. Thus panty liners or ultra-thin sanitary napkins also use cotton or viscose topsheets but target a limited consumer segment willing to accept the performance limitation for larger liquid gush volumes. So far cotton and viscose topsheet materials are not used in incontinence products which have to handle much larger amounts of liquid resulting in a too poor acquisition, also referred to as liquid intake or reception, and rewet performance of cotton and viscose topsheet materials.
However, in an attempt to improve this performance of the topsheet materials, it has been found that it is not sufficient to use naturally hydrophobic fibers like unbleached cotton or to treat the surface of the plant based fibers with a hydrophobic agent, as then then the inner structure of the plant based fibers still remains hydrophilic, thusly can absorb liquid and release this liquid, e.g. when submitted to pressure. Thus, it is desirable that the hydrophobization of such natural fibers penetrates into the inner of the fiber.
A particular approach to this is known from Kelheim Fibers, Germany, and available under the trade designation OLEA, as referred to in the above. It is based on regenerated cellulose as made by the viscose process, however a hydrophobic additive is penetrating into the fiber structure thusly not only rendering the surface hydrophobic, but also the core of the fiber. Such an additive is preferably selected from the group consisting of alkylketene dimers, alkenylketene dimers, alkylsuccinic anhydrides, alkenylsuccinic anhydrides, alkylglutaric anhydrides, alkenylglutaric anhydrides, alkyl isocyanates, alkenyl isocyanates, fatty acid anhydrides and mixtures.

However, it further has been found that such a hydrophobization improves rewet performance, but still provides insufficient acquisition performance. Mixing such hydrophobic fibers with higher amounts, i.e. more than about 10 w-%, or more than about 15 w-%, of hydrophilic fibers such as viscose or bleached or unbleached cotton reverts this effect and improves acquisition but deteriorates rewet significantly.

Thus, the present invention provides a solution to this dilemma by providing a hydroentangled web with internally hydrophobized fiber with apertures.
Referring to Fig. 2, the apertures of the web 120 may form a pattern 130 of apertures 122 within a land area 121. Within a pattern, the apertures 122 are preferably aligned along lines, as may be a row and column pattern, as shown in the figure, which may be positioned in a staggered arrangement, such that apertures 122 of neighbouring rows may be connected by a line angled by angle 139 to the length or machine direction 12 of the web. Preferably, such apertures exhibit an elongated shape, with a ratio of the longer dimension 125 to the shorter dimension 127 of more than about 1.5, or more than about 2.0, or more than about 3.0. The smallest extension 127 of the apertures may preferably be more than about 0.25 mm, or more than about 0.5 mm, or more than about 1 mm. The longer dimension 125 may be more than about 1 mm, or more than about 1.5 mm, or more than about 2 mm, or more than about 3 mm. The axes of the apertures may be aligned with the MD/CD orientation of the web, or may exhibit an angle thereto.
The apertures may exhibit an Effective Aperture Area, as may be determined according to the Aperture Test as described herein below, of more than about 0.1 mm², or more than about 0.3 mm², or more than about 0.4 mm², or more than about 0.5 mm² or more than about 1 mm². The Effective Aperture Area may be less than about 15 mm² or less than about 14 mm², or less than about 12 mm², or less than about 10 mm², or less than about 8 mm²m or less than about 5 mm².
Optionally, the apertures of the web may be arranged in sub-patterns exhibiting different Effective Aperture Areas, exhibiting a Relative Standard Deviation ("RSD") of the sub-patterns of at least about 20 %, or at least about 30 %, or at least about 50 % or at least about 55 %, or at least about 60 %, relative to the larger of the values.
A hydroentangled web of the present invention may have an Effective Open Area as the sum of the aperture areas relative to the land area, for a certain portion of the web, as may be determined according to the Aperture Test as described herein below of more than about 1 %, or more than about 5 %, or more than about 8 %, or more than about 8 %, or more than about 15%, and less than about 50 %, or less than about 40 %, or less than about 35 %, or less than about 30 %, or less than about 25 %, or less than about 15 %.
A hydroentangled web may have apertures that have an Average Interaperture Distance as may be determined according to the Aperture Test as described herein below along the x-direction 12 of the web (131), or along the width direction 18 of the web (132) of less than about 20 mm, or less than about 15 mm, or less than about 11 mm, or less than about 10 mm, or less than about 8 mm, or less than about 6 mm, or less than about 5 mm, or less than about 4 mm or less than about 3.5 mm, or less than about 3 mm, or less than about 2.5 mm, or less than about 2 mm, or less than about 1.5 mm, or less than about 1 mm. The range of the Average Interaperture Distance may preferably be of about 1 mm to about 6 mm, or of about 1 mm to about 5 mm, or of about 1 mm to about 4 mm, or of about 1 mm to about 3.5 mm, or about 1 mm to about 3 mm, or of about 1 mm to about 2.5 mm, or of about 2 mm to about 4 mm, or of about 3.5 mm to about 10 mm, or about 0.08 mm to about 11 mm.
The aperture pattern of the hydroentangled web may exhibit sub patterns, which may exhibit differences of aperture shape, size or orientation, or Effective Aperture Area or Interaperture Distances.
Such apertures have been described and may suitably be formed during the hydroentangling process, also interchangeably referred to as spunlacing process, by replacing the conventional hydroentangling screen by a suitably patterned screen. However, it is within the scope of the present invention that a homogeneous web may be formed and the apertures may be formed by other conventional means such as punching, or slitting with subsequent stretching, in particular cross-directionally.
Suitable webs exhibit a hydrophobicity as may be determined by the water Single Load Drop Test, as described herein below, wherein for a hydrophobic web at least three drops, as applied to the web, regardless if in a land region or also covering an aperture, remain on the surface for at least 60 secs.
Such a hydrophobicity can also be determined by submitting webs to the run-off test, as described herein below, wherein a suitable web retains less than about 10 w-% of test liquid based on the dry weight of the web, or less than about 7 w-% or less than about 5 w-%. For certain applications, it may be desirable to even further enhance the initial wetting of the material, preferably also improving the acquisition time, without compromising o the rewet performance. In a first approach, this may be addressed by adding a minor amount of hydrophilic fibers, such as less than about 10 w-%, or less than about 5 w-%, of e.g. cotton fibers not treated with an in-fiber hydrophobizer. In another approach, the surface of the web of hydrophobic fibres may be treated with small amounts of hydrophilizing agent, such as surfactants that readily wash off the surface. This provides the effect that at an initial loading the wetting is improved without detrimentally impacting rewet.
Such webs may very suitably be incorporated in absorbent articles, that exhibit a particular balance of good rewet performance and good acquisition performance for medium to high liquid loads according to the acquisition / rewet test as described herein below, preferably at a performance level as known from conventional articles not comprising natural material based topsheets. Such articles preferably exhibit a rewet value of less than about 3 g, or less than about 2 g, or less than about 1.5 g, or less than 1.0 g whilst exhibiting a cumulated acquisition time of less than about 400 sec, or less than about 350 sec, or less than about 300 sec, or less than about 250 sec, or less than bout 200 sec.
A skilled person will realize that such performance data are not only dependent on the properties of the topsheet, but also are impacted by the underlying layers. Thus, when webs according to present invention comprising suitable apertures are compared to equivalent webs without apertures over otherwise comparable elements such as ADL and core, the rewet or the acquisition time, preferably both, are significantly improved.

### Exemplary execution

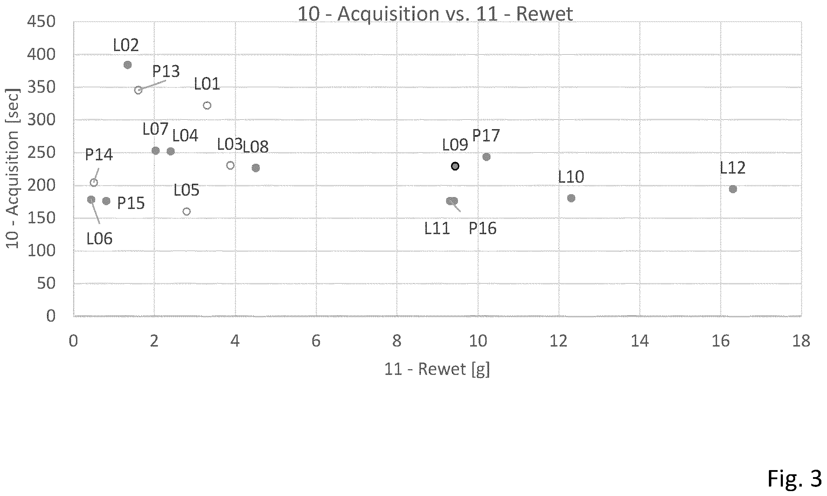

In order to further explain the present invention, laboratory testing was executed by testing various test specimen as such and on modified absorbent articles according to the test methods as described herein below. Details of the tested products and as well as respective test results are shown in Table 1 and further visualized in Fig. 3, plotting cumulated acquisition times in seconds (10) versus rewet values in gram (11). Executions according to the present invention are indicated by the suffix "#".
The basis for testing the absorbent articles was an adult incontinence pad SIEMPRE EXTRA, commercially available in Germany and intended to be used for high urine loads (marked up on package with "four drops" out of five). The pad comprises a core comprising NSKK fluff pulp and superabsorbent polymer particles, exhibiting a Free Swell Absorbent Capacity of about 520 ml and a Centrifuge Absorbency of about 185 g. An acquisition/distribution layer (ADL) is a polyester through air bonded web of about 50 g/m², commercially available from TWE, Germany under the trade designation DRYWEB T28, hereinafter referred to as A0. The topsheet of the commercial product is an about 18 g/m² carded non-permanently hydrophilized polypropylene topsheet hereinafter referred to as T0, commercially available Pely-tex GmbH, Germany, under the trade name ppH18. A first set of products, marked with an "L", was modified by removing the ADL and topsheet by using ice-spray, such as comprising a propane - butane mixture, so as to deactivate adhesives and allow replacement by test materials.
A second set of products was manufactured on a commercial production equipment, marked with a "P". Thus, sample L06 represents the modification effect as a modified sample with the materials as of the commercial production line products P15.
As a further comparative material, a topsheet material T1-100 was a carded air through bonded web of 35 g/m² made of 100% modified regenerated cellulose fibers at a fiber thickness of about 1.7 dtex, commercially available under the trade name OLEA by Kelheim Fibres, Germany, and carded in analogy to T0. The web exhibited a homogeneous fiber distribution typical for conventional carded product.
Variants of T1 were made by replacing the OLEAs fibers partly by viscose fibres of a thickness of 1.7 dtex as available from Kelheim Fibres, Germany. These variants were T1-85, T1-70, and T1-50, with respective percentage of OLEA fibers. All T1 samples were hydrophobic according to the Single Load Drop Test.
Further comparative samples were
- T2 as a 35 g/m² hydroentangled web of 100 w-% bleached cotton fibres,
- T3 as a 35 g/m² hydroentangled web of 80 w-% unbleached cotton fibres and
   20 w-% bleached cotton fibres,
all produced in analogy to T0.
Further samples suitable to be used in the current invention, marked as T*, were produced in analogy to T1 to T3, but have been submitted to a hydroentangling step, which induced a pattern of perforation, exhibiting an elongated shape with a length of about 1.7 +/- 0.3 mm in machine direction (MD) and a width of about 0.5 +/- 0.1 mm cross-directionally (CD). In MD, the "land area" between the apertures was about 1.5 +/- 0.2 mm, in CD 0.7+/- 0.1 mm, such that there were about 3 openings per cm in MD, and about 8 openings per cm in CD. The apertures of adjacent rows were staggered, such that cross-directionally the next neighbour of an aperture was separated by a land area of about 1.9 mm

Such topsheet materials were tested in combination with different commercially available underlying ADL, namely
A1: Spunlace nonwoven, 54 g/m², 0.81 mm thickness, 80% PET fibers, 20% viscose, fibers at a diameter of about 22 µm.
A2: Spunlace nonwoven, 51 g/m², 0.58 mm thickness, 65% PET, 20% viscose, 15% bicocomponent PE/PP fibers, fiber diameter of about 19 µm.
With these materials, the following test samples have been made and submitted to the acquisition and rewet test. Whilst samples L1 to L12 have been made by manual modifications in the laboratory, samples P13 to P17 have been made on a commercial manufacturing line.

**Table 1**

| Reference # | Topsheet | ADL | Rewet (g) | cumulated acquisition time (sec) |
|---|---|---|---|---|
| L= Laboratory | | | | |
| P = Production | | | | |
| L01 | T1*-100 | A0 | 3.3 | 322 |
| L02 | T1-100 | A2 | 1.33 | 384 |
| L03 | T1*-85 | A0 | 3.87 | 230 |
| L04 | T1-100 | A2 | 2.4 | 252 |
| L05 | T1*-100 | A2 | 2.8 | 160 |
| L06 | T0 | A0 | 0.43 | 178 |
| L07 | T1-100 | A0 | 2.03 | 253 |
| L08 | T1-85 | A0 | 4.5 | 226 |
| L09 | T1-70 | A0 | 9.43 | 229 |
| L10 | T1-50 | A0 | 12.3 | 180 |
| L11 | T2 | A0 | 9.3 | 176 |
| L12 | T1-50 | A1 | 16.3 | 194 |
| P13 | T1*-100 | A0 | 1.6 | 345 |
| P14 | T1*-100 | A1 | 0.5 | 204 |
| P15 | T0 | A0 | 0.8 | 176 |
| P16 | T3 | A0 | 9.4 | 176 |
| P17 | T4 | A0 | 10.2 | 243 |

In these tests, samples L06, P15 represent the reference of current products with synthetic materials T0 and A0, whilst samples L11 and P16 with pure bleached cotton T2, or P17 with a blend of 80 w-% unbleached and 20 w-% bleached cotton T3 represent common approaches of employing cotton materials with unsatisfactory balance of rewet and acquisition performance.

Samples L04 and L07 that were made using a topsheet T1-100, show a moderate acquisition time and good rewet results over ADL A0. Without wishing to be bound by the explanation, it is believed that the test set up with a water column in the test cylinder induces sufficient hydrostatic pressure on the topsheet resulting in a pressure driven wetting of the topsheet by filling the inter-fiber pores and making it water permeable. However, it should be noted that this not necessarily reflects real use conditions, especially for incontinence products, as urine may get in touch with the topsheet without application of pressure, resulting in liquid moving on the surface of the topsheet ("run-off') instead of penetrating it.
For topsheet samples with an increasing amount of viscose fibres being added to the OLEA fibers, see L07 to L10, the hydrophilicity of the surface is increased and thus the liquid acquisition improved, but the rewet is significantly deteriorated.
This underlines that rewet and acquisition time are competing parameters if viscose and OLEA fibres are blended in a such a spunlace material. Just blending Olea fibres and viscose fibres does not allow to design a topsheet suitable for use in incontinence products due to the compromise of rewet and acquisition time.

Increasing the hydrophilicity of the ADL by replacing A0 by a more hydrophilic ADL does not improve the speed of liquid acquisition but even has a negative effect on the rewet, see sample L12 with ADL A1, or sample L02 with ADL A2.

Thus, a topsheet T1-100 made of 100% Olea fibers could provide a good rewet similar to products using a conventional PP topsheet, however liquid acquisition would be strongly compromised - as might be improved by adding larger amounts of hydrophilic viscose fibres in order to achieve a spontaneous wetting and to achieve a speed of liquid acquisition competitive to conventional absorbent products using a PP topsheet with hydrophilic treatment, which however results in significant deteriorated rewet performance.
Without wishing to be bound by the theory, a spunlaced topsheet with 100% Olea fibres is facing the dilemma of not providing both good rewet and acquisition:
a) First, it is not spontaneously wettable and requires a certain pressure to fill the pores with liquid before liquid can flow across the material into the acquisition layer to the core. This will result in liquid run-off on the surface resulting in leakage of the product.
b) Second, driven by the poor wetting of the 100% Olea fibre topsheet the acquisition time of incontinence products using such topsheet material is significantly longer compared to conventional PP (hydrophilic) topsheet materials. As the barrier is in penetration of the topsheet using an acquisition layer with higher capillary force does not solve this issue.

This dilemma is resolved by using T1*-100, the apertured 100% Olea fiber based spunlaced web exhibiting exhibited a thickness of about 0.45 mm. as was tested with samples produced by replacing the topsheet of the absorbent product and replacing it by a test material, see samples L01, P13. Adding 15% of hydrophilic viscose fibers in the topsheet T1*-85, see sample L03, improves acquisition time without deteriorating rewet significantly. This effect is further enhanced by the combination in sample P14 with a spunlace ADL A1 containing viscose fibers (80% PET / 20% viscose).

In a further test, the run-off of a material T1*-100 was compared to a conventional hydrophobic polypropylene topsheet of 33 g/m² in the modified run-off test, as described herein below. Both webs exhibited very comparable results of about 22.4 g of liquid retained in the sample.
A further test, the effect of hydrophilization of the surface of a hydrophobic web was demonstrated by the repeated strike-through test over comparable absorption material, as described herein below. The comparable absorption material was the Siempre extra product, as referred to in the above, with ADL removed and the topsheet replaced by
- a standard hydrophilic 18 g/m² topsheet T0, as described in the above;
- the perforated topsheet T1*-100, as described in the above;
- the perforated topsheet T1*-100 treated with a surfactant;
- the perforated topsheet T1*-100 treated with a fragrance solution.
The surfactant was commercially available from SEDAC Chemie GmbH, Germany, under the designation Handspülmittel lemon - Sedac, comprising about 10% anionic surfactants, diluted with 99w-% of water. It was applied by a single puff of a cosmetic hand-spray pump, for which one puff provided approximately 0.2 ml of solution.
The fragrance solution was prepared by diluting 20 w-% of fragrance oil (aloysia citrodora) with 0.5 w-% of dried aloe vera and 79.5 w-% water.
Both the surfactant and the fragrance solution were applied by a single puff of a cosmetic hand-spray pump, for which one puff provided approximately 0.2 ml of solution.

**Table 2**

| strikethrough time in secs | Topsheet T0 | Topsheet T1*-100 | Topsheet T1*-100 + surfactant | Topsheet T1*-100 + fragrance oil |
|---|---|---|---|---|
| 1^{st} load | about 5 | 213 | 9 | 7 |
| 2^{nd} load | about 5 | 72 | 9 | n.a. |
| 3^{rd} load | 11 | 25 | 13 | 10 |

The results of table 2 demonstrate that non-permanent treatment of the otherwise permanently hydrophobic topsheet significantly improves wetting of the material.

### Test methods

All testing should be performed in a conditioned room maintained at about 23 +/- 2 °C and about 50 +/- 2 % relative humidity.

### Aperture measurements

The geometric determination of the apertures and their patterns may be determined by any suitable optical method. A particular suitable method may be employed according to the disclosure in EP3215086, to which express reference is made for the aperture related aspects of the "Aperture / Feret Angle Test", wherein aperture dimensions, effective open area and inter-aperture distance measurements are obtained from specimen images acquired using a flatbed scanner using a reflectance mode at a resolution of 6400 dpi and 8 bit grayscale. The scanner is interfaced with a computer running an image analysis program, and calibrated against an acquired image of a ruler certified by NIST. The test specimen may be mounted in a steel frame and be backed with a black glass tile.

### Free Swell Absorption Capacity

Following the Test Method PA07/05 of Hygiene-Technologie GmbH (Hy-Tec), Haan, Germany, the test specimen is submersed in sufficient 0.9 w-% saline for 30 mins, after which is removed and placed on a coarse grid or sieve, e.g. a grid with 1 cm wide opening and a ribs of about 2 mm. After a further waiting time of 10 mins, the weight of the test specimen is recorded as free swell absorbency in grams-.

### Centrifuge Capacity

This test is generally following the EDANA NWSP 241.0.R2 (15) Polyacrylate Superabsorbent Powders - Determination of the Fluid Retention Capacity in Saline Solution by Gravimetric Measurement Following Centrifugation, but applying it to an absorbent article, as per Test Method PA07/05 of Hygiene-Technologie GmbH (Hy-Tec), Haan, Germany. To this end, a test sample as treated according to the free swell absorption test is positioned within a commercial centrifuge exerting a centrifugal force of about 250 times gravity (e.g. by having an internal diameter of 225 mm at a rotation of 1400 rpm) for 3 mins. The test specimen is removed and weighed and the centrifuge absorbent capacity recorded in grams.

### Acquisition & Rewet Determination

The Acquisition / Rewet measurement is based on the Nonwoven Adult Incontinence products: Rate of Acquisition and Re-Wet Test (EDANA Test method NWSP 070.9.R1 (15)) however modified according to the Test Method PA07/03 of Hygiene-Technologie GmbH (Hy-Tec), Haan, Germany.
The test employs an apertured Perspex plate of 70 mm by 220 mm with a central cylinder of an inner diameter of 20 mm and a height of 130 mm at a weight of 255 g, adapted such that weights of 2 kg each can be placed on onto the plate on either side of the cylinder. Upon placing the cylinder and the plate with weights centered on the plate, 50 ml of 0.9 w-% saline as synthetic urine are applied to the cylinder, and the acquisition time until complete disappearance of the liquid is recorded as 1^{st} gush acquisition time. 20 mins after that point in time, the second 50 ml are applied, and the 2^{nd} gush acquisition time until disappearance is recorded. After another 20 mins, the 3^{rd} 50 ml are applied and the 3^{rd} acquisition time is recorded. After 5 mins, the weights, plate and cylinder are removed and accurately (i.e. +/- 0.01 g or better) weighed 50 g of filter paper of 110 mm by 230 mm, are placed on the test specimen, followed directly by applying a plate without cylinder at 145 g and the 2*2 kg weights. After 15 secs, the weights and plate are removed and the rewetted filter paper is accurately weighed. The three acquisition times are added to the cumulative acquisition time in seconds, and the rewet value is recorded in grams.

### Single Load Drop Test

At least three drops of de-ionized water / saline, for which contamination by any surface active substance has been avoided, thus exhibiting a surface tension of at least 70 dyn / cm, are placed on a test specimen, such as by a clean pipette. By visual observation it is determined, if these drops are penetrating into the substrate or remain on the surface for at least 60 secs. In the latter case, the test specimen is considered hydrophobic, otherwise hydrophilic.

### Washed Substrate Drop Test

In order to assess the performance of hydrophobic topsheets that received a non-permanent hydrophilization treatment, such that these materials are hydrophilic according to the Single Load Drop Test, such webs are washed for about 1 min in an excess of water at about 30°C and dried under ambient for at least 24 hrs. The Single Load Drop Test is the repeated, and in case the drops remain on the surface, the web is considered "only initially hydrophilic", otherwise "permanently hydrophilic".

### Topsheet Run-off Test

This test aims at determining the ability of the material to retain liquid applied thereto. This test is based on the EDANA test method Nonwoven Coverstock Run-Off test NWSP 080.9R0 (15), and employs a 10° inclined base plate onto which the test specimen was placed at a length of at least 25 cm extending from the lower end of the plate. At that point 25 ± 0.5 g of 0.9 w% saline was applied through a centered tube with an inner diameter of 5 mm, distanced vertically 25 mm above the web, at a flow rate of 6.3 g/sec. After application of the fluid the weight increase of the web was recorded.

### Nonwoven Thickness

The thickness for the samples was determined generally following EDANA Standard Procedure NWSP 120.1.R0, with a circular pressure foot with 3 cm diameter at a pressure of 15g/m².

### Nonwoven Fiber Diameter

The diameter of the fibers in a nonwoven were optically determined under a microscope at a magnification of 200X and at least 6 measurements per sample.

## Claims

1. An absorbent article
comprising
a fibrous topsheet;
a backsheet;
and an absorbent core positioned there between
exhibiting an absorbent capacity of at least 150ml of synthetic urine according to the Centrifuge Capacity Test;
wherein said topsheet comprises at least about 90 w-%, preferably at least about 95 w-%, and even more preferably consists essentially of plant based fibers, preferably cellulose based, more preferably of regenerated cellulose;
wherein said fibers are hydrophobically treated by
incorporating a hydrophobic substance into the fiber matrix,
said hydrophobic substance preferably being selected from the group consisting of alkylketene dimers, alkenylketene dimers, alkylsuccinic anhydrides, alkenylsuccinic anhydrides, alkylglutaric anhydrides, alkenylglutaric anhydrides, alkyl isocyanates, alkenyl isocyanates, fatty acid anhydrides and mixtures thereof;
wherein said topsheet exhibits land area and a pattern, optionally comprising sub-patterns, of apertures,
said apertures exhibiting at least one of the following features selected from the group consisting of
- the apertures exhibiting an elongated shape, wherein the longer extension is at least 1.5 times the smaller extension;
- the apertures exhibiting a smaller extension of at least about 0.25 mm;
- the apertures exhibiting a lager extension of at least about 1.00 mm;
- the aperture area being at least about 0. 1 mm²;
- the aperture area being less than about 15.0 mm²,
- the apertures covering at least about 1 % of the total area of said topsheet;
- the apertures exhibiting covering less than about 50% of the total area of said topsheet;
- an average Interaperture distance of more than about 1mm;
- an average Interaperture distance of less than about 20 mm;
wherein said topsheet further exhibits a hydrophobicity according to the Single Load Drop Test.

2. An absorbent article according to claim 1, wherein
said regenerated cellulose is based on cotton.

3. An absorbent article according to any of the preceding claims, wherein
said regenerated cellulose is based on the lyocell process.

4. An absorbent article according to any of the preceding claims, wherein
said topsheet further comprises hydrophilic fibers, preferably regenerated cellulose fibers, in an amount of less than about 10 w-%, preferably less than about 5 w-%.

5. An absorbent article according to any of the preceding claims, wherein
said topsheet comprises a non-permanent hydrophilization agent.

6. An absorbent article according to any of the preceding claims, wherein
said topsheet comprises a fragrance solution.

7. An absorbent article according to any of claims 5 to7, wherein
said topsheet exhibits only an initial hydrophilicity and not a permanent hydrophilicity according to the Washed Substrate Drop Test.

8. An absorbent article according to any of the preceding claims,
exhibiting a rewet of less than about 10 g, preferably less than about 5 g, and more preferably less than about 3 g, and most preferably of less than about 2 g. whilst exhibiting an acquisition time of less than about 200 s, according to the acquisition and rewet test using 3 x 150 ml of 0.9 % saline solution.
